# EUROPEAN PATENT SPECIFICATION

(11) **EP 4 190 240 B1**
(45) Date of publication and mention of the grant of the patent: **02.09.2026**
(21) Application number: 21383091.2
(22) Date of filing: 03.12.2021
(51) Int. Cl.: A61B 5/00, A61B 5/266, A61B 5/27, A61B 5/271, A61B 5/291

(54) **AN ELECTROENCEPHALOGRAM MEASUREMENT DEVICE AND A PROCESS FOR MANUFACTURING THEREOF**
VORRICHTUNG ZUR MESSUNG EINES ELEKTROENZEPHALOGRAMMS UND VERFAHREN ZU DEREN HERSTELLUNG
DISPOSITIF DE MESURE D'ÉLECTROENCÉPHALOGRAMME ET SON PROCÉDÉ DE FABRICATION

(43) Date of publication of application: 07.06.2023
(73) Proprietor: Bit&Brain Technologies, S.L., 50008 Zaragoza (ES)
(72) Inventor: López Larraz, Eduardo, Zaragoza (ES); Moreno Cantón, Jorge, Zaragoza (ES); Ferri Pascual, Josué, Zaragoza (ES)
(74) Representative: Clarke, Modet y Cía., S.L.

(56) References cited:
- WO-A1-2021/214433
- CN-A- 113 558 627
- US-A1- 2017 196 514
- US-A1- 2020 237 248

## Description

### Field of the invention

The present invention refers to a device for measuring electrical brain activity, also known as electroencephalogram measurement device or EEG (electroencephalography) device, whose sensor layer is made only with threads (including yarns) and/or textiles (including fabrics) to form a garment, or to be integrated into an existing garment such as a cap or headband, or integrated in any other type of device not made of textiles such as a plastic neuroheadset or a virtual reality system.

The present invention also refers to a process for manufacturing an electroencephalogram measurement device whose sensor layer is partially or integrally made with threads and/or textiles.

### Background of the invention

An EEG device is generally composed by two parts: the sensor layer and the amplifier. On the one hand, EEG sensor layer is made by a biopotential measurement sensor or electrode that contacts the scalp of a user for measuring EEG signals from said user, a transmission cable that transports the EEG signals from the electrode to an electronic signal amplifier, and means to attach the electrodes and the transmission cables to a support in the head in the form of a headset. On the other hand, the EEG system further comprises the amplifier that amplifies and digitizes the electrical EEG signals measured by the electrodes. Usually, the amplifier also provides means to store or send the data to a computation unit. In some implementations there is an interface between the EEG sensor layer and the EEG amplifier denoted connector.

In modern EEG systems, the electrodes are usually made of metals, conductive polymers, or printed inks among other conductive materials; whereas the transmission means are usually cables, wires, printed inks, or PCBs usually shielded to protect the EEG signal from artefacts. This shielding allows the mitigation of noise from electromagnetic interference and mechanical noise generated by the movement of sensors or cables. These shielded cables or wires carry the EEG signals from the electrodes to the electronic signal amplifier, insulating said EEG signals from the electromagnetic, mechanical or other sources of noise. Shielding is of paramount importance to transport EEG signals due to the low signal-to-noise ratio of the EEG activity. Thanks to modern EEG transmission shielding, it is possible to perform high quality EEG tolerant to many sources of noise almost anywhere, without requiring shielded rooms or Faraday cages.

The electrodes and the transmission cables are normally anchored to a headset with a cap-like structure, which also enables the contact between the sensors and the skin/scalp on the subject's head.

It is denoted a wet-EEG device when an electrolytic substance is used between the electrode and the skin to increase conductivity and improve the impedance contact, and dry-EEG device when there is no need to any electrolytic substance, providing dry contact with the skin.

As an example of a wet-based EEG device, it is known a device that comprises a plurality of electrodes attached to a cap, similar to a "swimmingcap", with the EEG amplifier in a box attached to the back of the head. This EEG device needs the application of electrolytic gel or saline water to work. That is, it needs to add an electrolyte substance to improve the electrode-skin contact. Modern wet-EEG systems use shielded cables to transport the EEG signal to the signal amplifier to have high performance in terms of signal-to-noise ratio.

As an example of a dry-EEG device, it is known a device that comprises a plastic neuroheadset in which both the EEG sensor and amplifier layers are integrated into a plastic housing. This EEG device has silver metallic sensors able to operate in contact with the skin without conductive substances. This device uses shielded cables hidden within the plastic structure to transport the EEG signal to the signal amplifier. Shielded cables are much more important in dry-EEG than in wet-EEG devices. This is because dry sensors have higher impedance contact, and thus are prone to be affected by measurement noise and artefacts, leading to lower signal to noise ratios and thus data that cannot be interpreted later on.

In terms or materials, there is currently a great development trend around threads and textiles with electrical conductive properties, also known as smart textiles, for the development of garments with sensors. The main advantage of smart textiles is that they allow the implementation of wearable technologies with body measurement sensors seamlessly integrated in human garments or clothes, being almost transparent for the user. In addition, they are bendable, soft, and breathable, thus offering the user a greater comfort.

Smart textiles are based on measurement layers integrated into the garment. A set of these textiles are based on a technology that allows generating threads, fabric or textiles with silver content, or other conductive materials, by manufacturing or treatment. Said fabric, threads or textiles can be introduced in conventional weaving machines, and thus generate garments in which the conductive properties go completely unnoticed inside the garment (i.e., the electrode and the transmission are textiles that go unnoticed in the garment itself). These garments are normally plugged into an electronic system that performs the electrical signal acquisition and digitization, also denoted as signal acquisition amplifier.

There are garments based on smart textiles able to measure physiological variables such as ECG (electrocardiogram), EMG (electromyogram), EOG (electrooculogram), etc., which are characterized in the millivolt range. However, the measurement of EEG (electroencephalograms), characterized in the microvolt range, has not been successfully achieved yet implemented only with threads or textiles. This is because of the technical difficulties of dealing with low signal-to-noise ratios derived from very high impedance electrode-skin contact and poorly shielded signal transmissions. That is, EEG signals are at a much lower scale and thus are prone to be easily contaminated by noise, which are originated if there are poor impedances, unstable electrode-skin contacts, or ineffective shielding in the signal transmission.

Therefore, the existence of garment designs integrally manufactured with threads or textiles and that can be used as sensors layers to measure with accuracy and precision the electroencephalogram (EEG) is unknown. Likewise, the existence of EEG devices comprising electrodes and shielded transmissions only made of threads and/or textiles encapsulated on headsets is also unknown. When the headsets are made of textiles then the full EEG sensor layer is a garment.

Documents US2017/196514A1 and WO2021/214433A1 disclose other devices that disclose the technical features of the preamble of claim 1.

The present invention refers to an effective EEG device comprising EEG sensor layers completely or partially implemented with threads (including yarns) and/or textiles (including textile fabrics). The fact that the sensor layer, or their main parts, which are the electrodes and transmissions, are made of textile provides the following advantages over traditional EEG devices:
- Acceptability: garments are naturally worn by people in all cultures.
- User comfort: it is not implemented with metal-based electrodes and cables, which are uncomfortable for long-time use and constrain user mobility. In addition, they are lighter than other systems, and can be integrated in existing devices (such as headsets or virtual reality glasses) or garments naturally worn making them more comfortable.
- Adaptability to the user: compared to equipment with plastic housings, fabric/textile equipment has the peculiarity that it can be designed to adapt and accommodate to the shape of the head, thus being more comfortable.
- Cleaning maintenance: conductive substances such as electrolytic gels are not required, and sensors layers based on textiles can be naturally washed.
- Reduction of artifacts: electromechanical noise due to the movement of wires is eliminated (no wires), allowing a higher signal-to-noise ratio. This allows the equipment to be used in applications where the user moves freely.
- Reduction of sweat artifacts: the textiles that form the garment can be chosen or treated to be breathable, so that the person does not sweat (eliminating this artifact).
- Adaptability to the application: the textiles that form the garment can be chosen or treated to have anti-bacterial, anti-odor properties (ostensibly improving hygiene and cleanliness), and many other options that make the final EEG device more adapted to the final application.
- Ease of use: the garments (specially the caps) have no preparation time (as opposed to conventional EEG systems that have to be carefully placed, adjusting every single electrode, adding the conductive substances, etc.).
- Costs: the cost of a piece of fabric/textile is much lower than metals, cables, plastics due to the costs of the raw materials, number of providers, number of industries involved, implementation of quality processes, etc.
- Increased sensor density: current sensor technology has the limitation that the electrode assembly, sensor base, solder and cable cannot be miniaturized, and furthermore when applying conductive substances, they can produce short-circuits. In fact, the devices with the largest number of sensors have 256 (denoted high density EEG devices). Fabrics do not have this limitation and can be miniaturized as much as desired, given that the entire cap is made of the same material (fabric).
- Maintenance: they do not have, as opposed to traditional sensors and cables that wear out or break.

The present invention also refers to a process for manufacturing an EEG sensor layer of an EEG device, which is integrally or partially made with threads and/or textiles. Thus, being much easier and faster to implement, since electrical, electronic and mechanical components of the EEG amplifier such as metals, solder, cables, plastics for support, etc. are completely eliminated. In addition, this process allows manufacturing the EEG device to integrate said device in an existing device (such as a headset or virtual reality glasses) or garment, or manufacturing the EEG device together with the garment, that is, at the same time.

### Description of the invention

The dry electroencephalogram measurement device of the present invention comprises a head support on which the following components are attached and/or integrated:
- at least one electrode, also called sensor, for measuring EEG signals from a user; and
- at least one shielded cable, electrically connected to the at least one electrode, for transporting said EEG signals to an electrical signal amplifier, and/or for transporting other control signals from a stimulator to at least one electrode (for example, a stimulation electrode).

The support, the at least one electrode and the at least one shielded cable are integrally made with threads and/or textiles. The sensor layer is thus made integrally of threads and/or textiles.

The support allows the attachment and/or integration of the at least one electrode and the at least one shielded cable, and it may adopt many different configurations, mostly acting as a head support. For example, the support can be directly an object, such as a garment, apparatus, or device; a part of the same; or a separate or independent element, such as a textile fabric, etc. specially intended to be attached or integrated onto a surface of said object, apparatus or device, or part of the same.

The support can be made of threads and/or textiles, for example a Lycra^{®} knitted fabric, combined or not with other materials such as plastics or aluminium. For example, a garment, such as a headband, a cap, a fabric-covered diadem, a semirigid hairpin covered with fabric, etc.

In general, the support is preferably selected, designed and/or manufactured considering different aspects, such as washability requirements, sizes, scale up and industrialization processes, etc. When the support is directly in contact with the skin of the user, is preferably made of a soft and comfortable/warm material to be in use during some hours, also having a good performance as anti-bacterial to stay fresh for longer. The material for the support is also preferably flexible and elastic to facilitate the contact with sensors on the skin in a dedicated way. All these properties can be selected for the materials forming the support, in such a way that they better fit the requirements of the application.

The threads and/or textiles for the electrodes can be made with natural fibers, synthetic fibers, yarns or even fabrics coating the surface with metals, conductive threads produced with mechanical process or adding conductive particles to polymeric fibers. These conductive materials can be aluminium, carbon, copper, nickel or silver among others. It is noted that these sensors will be in touch with the skin of the user. So, in order to give comfort to the user, the mentioned materials for the conductive threads and/or textiles have been considered as they are suitable for use on the skin, and at the same time they have good conductive performance to obtain the desired electrical signals of the brain.

According to a preferred embodiment, the electrodes are made of conductive threads and/or textiles embroidered or sewn to the support. The electrodes are thus made of conductive threads and/or textiles directly embroidered or sewn to the support, for example a fabric, in the desired shape. So, this allows the design of patterns with different densities and shapes to be embroidered or sewn.

According to another preferred embodiment, the electrodes are made of conductive threads and/or textiles attached to the support by heat sealing with thermoplastics polymers, such as polyvinyl chloride (PVC) or polyurethane (PU), among others, or attached by other fixing means. The material forming the electrodes is thus shaped and cut to give it the desired form, and then integrated in the support, for example a plastic or textile, by heat sealing or sealing with heat and pressure. The materials must not fray as it could cause interference or malfunctions. Different conductive materials can be used depending on the manufacturing processes, such as non-woven, knitted, or woven fabrics. These materials have a base that could be polyamide, polyester, among others with a conductive material plated as nickel, copper, or silver among others.

For both embodiments, the size of the electrodes depends on the signal to be obtained. The dimensions variate from some millimeters to 5 cm range. The shape of the electrodes can be round or squared, etc. depending on the needs.

According to a particular embodiment, the electrodes may have salient fibers configured to go through the hair of the user and touch the skin, to improve the acquisition of EEG signals from the user. These salient fibers may be implemented during the embroidery or sewing process in a simple manner.

The shielded cables allow data transmission from the electrodes to the electrical signal amplifier. The shielded cables are made of a combination of conductive and insulating threads and/or textiles, forming a multi-layered shielded cable of "laminated type". The shielded cables of the present invention may therefore be considered as transmission tracks.

The conductive threads and/or textiles for the shielded cables can be made with short fibers, metal fibers, natural fibers or synthetic fibers, that can be made with conductive materials or coated with conductive particles. These conductive materials can be copper, nickel or silver among others. The insulating threads and/or textiles for the shielded cables can be made of foam, neoprene, pet, polyester, polyurethane, polyamide, rubber, silicone, or other materials with good insulating properties.

The electrical connection between the electrodes and the shielded cables is made through the conductive materials composing the same, so that they are in contact to guarantee conductivity between them. The materials connecting with the electrodes must have good conductivity to transmit the signal obtained and should also have good electrical affinity to the electrodes. The end of the shielded cables may comprise standard connectors or are directly connected to the amplifier inputs.

Data transmission is therefore made without standard cables that could constrain the daily use of the system, thus being a non-invasive solution. At the same time, this solution must deal with good signal transmission, reducing noise or interferences. For this purpose, the proposed shielded cables comprise a multilayer design, wherein the different layers of conductive and insulating materials are intercalated to implement a shielding. With this multilayer structure a flattened cable is achieved, wherein the core can be a wire or a conductive track. This structure offers the benefits of a shielded coaxial cable but flat and more flexible. The EEG signals are transported from the electrodes to the amplifier, insulated from electrical and electromechanical noise or interferences by means of another conductive layer that protects said signals.

According to a preferred embodiment, the shielded cables are made of a combination of conductive and insulating threads and/or textiles embroidered or sewn to the support.

According to another preferred embodiment, the shielded cables are made of a combination of conductive and insulating threads and/or textiles attached to the support by heat sealing with thermoplastics polymers, such as polyvinyl chloride or polyurethane, or attached by other fixing means.

Preferably, the at least one electrode and/or the at least one shielded cable are made of non-woven fabrics, knitted fabrics, or woven fabrics.

Preferably, the at least one electrode and/or the at least one shielded cable comprise a base of polyamide or polyester, plated or coated with conductive materials such as nickel, copper, or silver, tin, carbon, conductive polymers or combinations, among others.

The support, the electrodes and the shielded cables are all of them integrally made with threads and/or textiles. Therefore, the electroencephalogram measurement device comprising these components is also integrally made with threads and/or textiles.

The electroencephalogram measurement device of the present invention may also be combined with electrolyte substances to further improve the electrode-skin contact. That is, the electrodes may comprise an electrolytic substance to improve the conductivity between said electrodes and the user head. This can be done, for example, wetting or moistening the electrodes with water, saline solutions or conductive gels.

The properties of the threads and/or textiles forming the electroencephalogram measurement device of the present invention are also adapted to the final application and/or requirements of the EEG device, in terms of perspiration, anti-bacterial properties, anti-odor, waterproof, etc.

The electroencephalogram measurement device of the present invention may be implemented as a:
- headband for EEG, with the device on the front and the amplifier on the back;
- headband to make an EOG-based eye-tracker;
- swimming-like cap or wearable cap with hundreds of sensors >> 256 (usable for people with very few hair in the head);
- baseball cap with hundreds of sensors >> 256 (usable for people with very few hair in the head);
- swimming-like cap or wearable cap with thread-like fabric protrusions to go through the hair;
- swimming-like cap or wearable cap with fabric protrusions to go through the hair;
- semi-rigid hairpin covered with fabric, with a support for attaching the amplifier;
- fabric-covered diadem or headband;
- fabric "hair picker" with a place to leave the amplifier;
- head net with protrusions of fabric to go through the hair and make contact;
- waterproof swimming cap to make EEG in situations where hair can get wet like rain or under water;
- piece of fabric with textile sensors to cover the surface in contact with the head of headphones; or
- piece of fabric with textile sensors to cover the surface in contact with the head of helmets, such as the ones used for protection in work environments or for motorbiking.

Likewise, the electroencephalogram measurement device of the present invention may be used within the fields of research, user applications such as brain-computer interaction, or interventional and therapeutic for the following applications:
- Sleep research: study of brain activity while users are sleeping.
- Neuroscience research: study of different brain mechanisms (e.g., event related potentials or oscillatory activity) in laboratory environments.
- Out-of-the-lab Neuroscience: study of different brain mechanisms (e.g., event related potentials or oscillatory activity) in natural and uncontrolled environments.
- Brain interfacing as an alternative channel of communication with daily-life objects (e.g., anticipation of breaking intentions during driving).
- Detection of surveillance, alternate and drowsiness states (e.g., during work, study, or driving).
- Monitoring of mental states during activities of daily living (e.g., fatigue, stress).
- Brain interfacing for home environment control (e.g., control of lights, or mental password).
- Brain computer interface for motor rehabilitation of patients with motor impairment (e.g., stroke or spinal cord injury).
- Brain computer interface for cognitive and emotional training (e.g., to prevent or treat cognitive decline, or to improve cognitive performance in students).
- Closed-loop brain-state dependent stimulation for brain disorder treatment.

The present invention also refers to a process for manufacturing a dry electroencephalogram measurement device, comprising the following steps:
a) providing or manufacturing a head support made of textiles;
b) attaching or integrating to said support at least one electrode made of threads and/or textiles, for measuring EEG signals from a user; and
c) attaching or integrating to said support at least one shielded cable made of threads and/or textiles, electrically connected to the at least one electrode, for transporting said EEG signals to an electrical signal amplifier;
wherein the step c) comprises:
- providing or manufacturing a multi-layered shielded cable of laminate type made of a combination of conductive and insulating threads and/or textiles to form the at least one shielded cable; and
- sewing or embroidering the multi-layered shielded cable to the support; or
- attaching the multi-layered shielded cable to the support by heat sealing with thermoplastics polymers, such as polyvinyl chloride or polyurethane.

The step a) comprises the options of:
- providing an existing support, such as a headset or VR glasses; or
- manufacturing said support while the EEG device is also being manufactured, for example, when the device is a garment, such as textile cap or headband, the support is manufactured together with said garment, as a part of the same.

The support can be made of textiles, combined or not with other materials such as plastics or aluminium.

Preferably, the step b) comprises:
- embroidering one or more conductive threads to the textile support to form the at least one electrode; or
- sewing a conductive textile to the textile support to form the at least one electrode; or
- attaching one or more conductive threads and/or textiles to the textile support by heat sealing with thermoplastics polymers, such as polyvinyl chloride or polyurethane, to form the at least one electrode.

Embroidery technology allows inserting electrically conductive and non-conductive yarns in a defined pattern onto a textile substrate, such as fabrics. The embroidery process uses top and bottom yarns, which are interlaced every time the needle punches the fabric substrate to create the embroidery stitches. During embroidery the substrate fabric frame is moved in "x" and "y" directions to create the desired pattern. There is a branch of embroidery that is based on three yarn systems and is called tailored wire placement. This technique is used for exact placement of functional wire material onto a textile substrate by having the other two, top and bottom yarns stitch it down.

### Brief description of the drawings

What follows is a very brief description of a series of drawings that aid in better understanding the invention, and which are expressly related to different embodiments of said invention that are presented by way of nonlimiting examples of the same.
Figure 1 represents a schematic diagram of the electroencephalogram measurement device of the present invention.
Figure 2 represents an electrode embroidered or sewn to a textile support, according to a preferred embodiment of the present invention.
Figure 3 represents two electrodes attached to a textile support, according to another preferred embodiment of the present invention.
Figure 4 represents an exploded view of one of the electrodes of Figure 3.
Figure 5 represents a schematic view of a multi-layered shielded cable of "laminate type", according to a preferred embodiment of the present invention.
Figure 6 represents an exploded view of the multi-layered shielded cable of Figure 5.
Figure 7 represents a perspective view of an electrode connected to the multi-layered shielded cable of Figure 5.
Figure 8 represents a schematic view of a multi-layered shielded cable of "coaxial type", according to a not claimed embodiment of the present invention.

### Detailed description of the invention

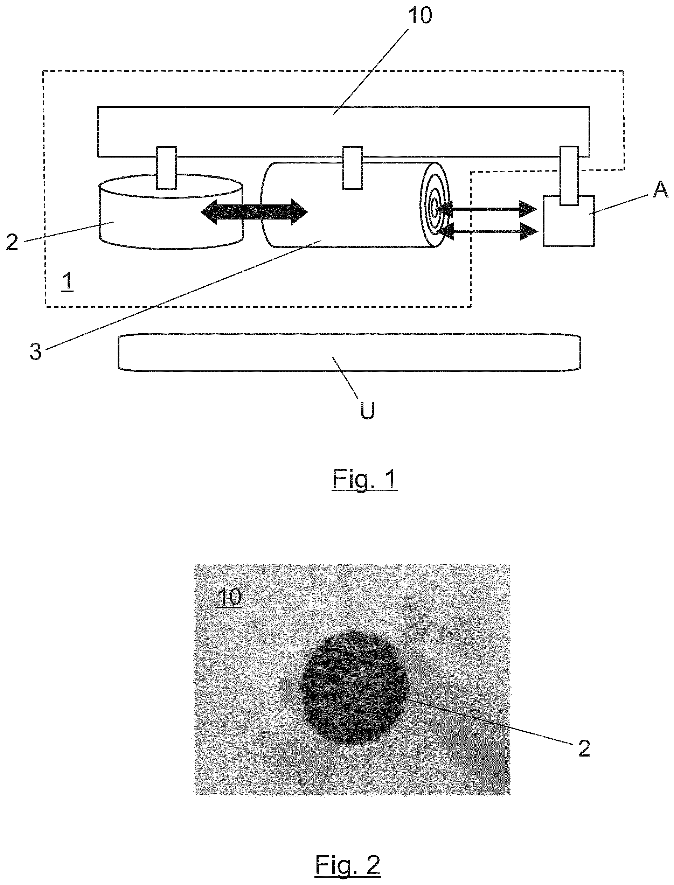

Figure 1 represents a schematic diagram of the electroencephalogram measurement device (1) of the present invention. As seen, it comprises a support (10) on which the following components are attached and/or integrated:
- at least one electrode (2) for measuring EEG signals from a user (U); and
- at least one shielded cable (3), electrically connected to the at least one electrode (2), for transporting said EEG signals to an electrical signal amplifier (A).

The at least one electrode (2) and the at least one shielded cable (3) are made of threads and/or textiles.

Figure 2 represents an electrode (2) made of conductive threads and/or textiles embroidered or sewn to a textile support (10), such as fabric. According to this example, the electrode (2) is round shaped.

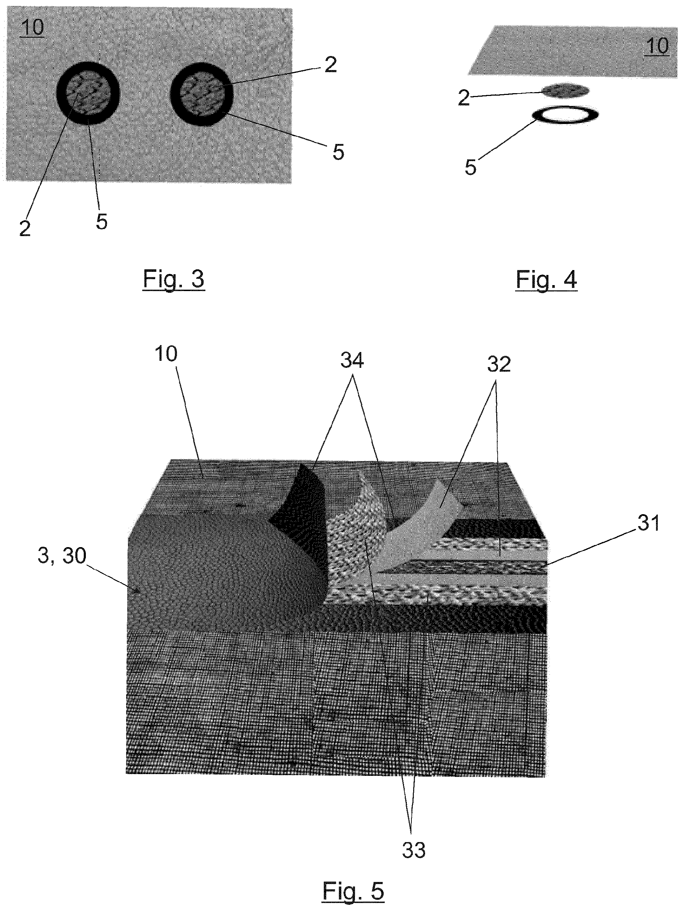

Figures 3 and 4 respectively represents two electrodes (2) attached to a textile support (10) and an exploded view of one of these electrodes (2), according to another preferred embodiment of the present invention.

As seen, the electrodes (2) are made of conductive threads and/or textiles attached to the support (10) by heat sealing with thermoplastics polymers (5), such as polyvinyl chloride (PVC) or polyurethane (PU), among others.

The material forming the electrodes (2) is thus shaped and cut to give it the desired form, and then integrated in the support (10). The conductive fabric cutting process can be carried out for instance with a plotter using a blade or laser. In the same manner, the thermoplastics polymers (5) can also be cut with a plotter.

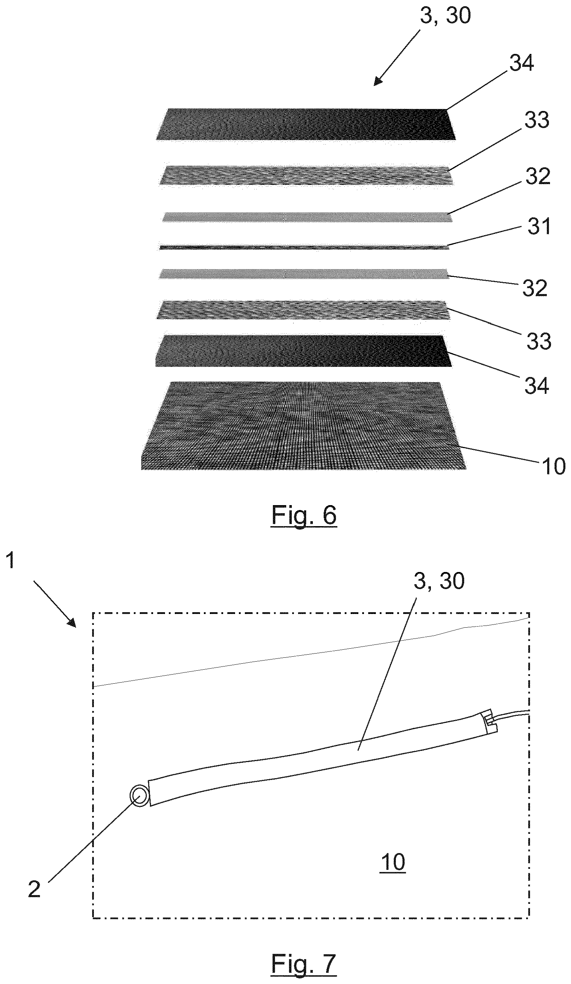

Figures 5 and 6 respectively represents a schematic view and an exploded view of a multi-layered shielded cable (3, 30) of "laminate type", according to a preferred embodiment of the present invention. According to this example, the multi-layered shielded cable (3, 30) is adequately integrated in a piece of clothing to transmit the electrical signals.

As seen, the multi-layered shielded cable (3, 30) comprises:
- one conductive layer (31) electrically connected to the at least one electrode (2), made of conductive threads and/or textiles, for transporting the EEG signals to the electrical signal amplifier (A);
- two inner insulating layers (32) between which the conductive layer (31) is disposed, made of insulating threads and/or textiles, non-conductive polymers, or other means used in the textile industry;
- two shielding layers (33) between which the inner insulating layers (32) are disposed, made of conductive threads and/or textiles, for protecting the EEG signals from electrical and electromechanical interferences; and
- two outer insulating layers (34) between which the shielding layers (33) are disposed, made of insulating threads and/or textiles.

The two inner insulating layers (32) may be formed by a single piece of fabric, folding the same to cover the conductive layer (31). In the same manner, the two shielding layers (33) may be formed by a single piece of fabric, folding the same to cover the inner insulating layers (32). And also, the two outer insulating layers (34) may be formed by a single piece of thermo-adhesive textile material, folding the same to cover the shielding layers (33).

According to this example, the conductive layer (31), the inner insulating layers (32), and the shielding layers (33) are made with fabrics. These fabrics have stretchable properties to allow deformation. On the other hand, the outer insulating layers (34) have a minimum thickness of 0.1 mm to correctly isolate the other layers.

According to a specific example, the different layers (31, 32, 33, 34) may each one of them have a 0.1 mm thickness approx. to have good flexibility, and a different width between them.

For the transmission layer corresponding to the conductive layer (31), the width may be 2 mm approx. The conductive layer (31) must have good conductivity to transmit the signal obtained and good electrical affinity with the electrodes (2).

The inner insulating layers (32) that cover the transmission may each have a width of 8 mm approximately, which allows them to isolate the transmission layer with a shielding layer. The inner insulating layers (32) are made of polyurethane to isolate the conductive layer (31) from the shielding layers (33). It needs to have good isolating properties.

The shielding layers (33) may each have a width of 10 mm approximately. The shielding layers (33) are composed by a top and a bottom layer which need to be in touch. This is the reason why the width of the shielding layers (33) is bigger than the width of the inner insulating layers (32). The shielding layers (33) do not need extreme conductivity, although they must be conductive.

Finally, the outer insulating layers (34) cover all the other layers (31, 32, 33), working as a protection for the rest of the layers. They are also used to attach the multi-layered shielded cable (3, 30) to the support (10).

The entire multilayer structure is compacted and sealed with pressure and heat to achieve a stable and homogeneous structure. In this way, the signal traveling through the conductive layer (31) is confined and protected against external interference.

According to this embodiment, the process for manufacturing an electroencephalogram measurement device of the present invention also comprises the following tasks:
- cutting the fabrics and thermo-adhesive textile material for the different layers (31, 32, 33, 34) with the shape and size specified for the final application of the device;
- assembling in the corresponding order the different layers (31, 32, 33, 34);
- applying temperature and pressure with a lamination process;
- embroidering the electrodes (2) with conductive threads; and
- sewing the whole device as textile band.

Figure 7 represents a perspective view of an electrode (2) connected to a multi-layered shielded cable (3, 30) of the "laminate" type, both integrated to the support (10). On the left side, the electrode (2) is connected to the conductive layer (31) of the multi-layered shielded cable (3, 30). On the right side, the multi-layered shielded cable (3, 30) is prepared to be plugged to the amplifier (A).

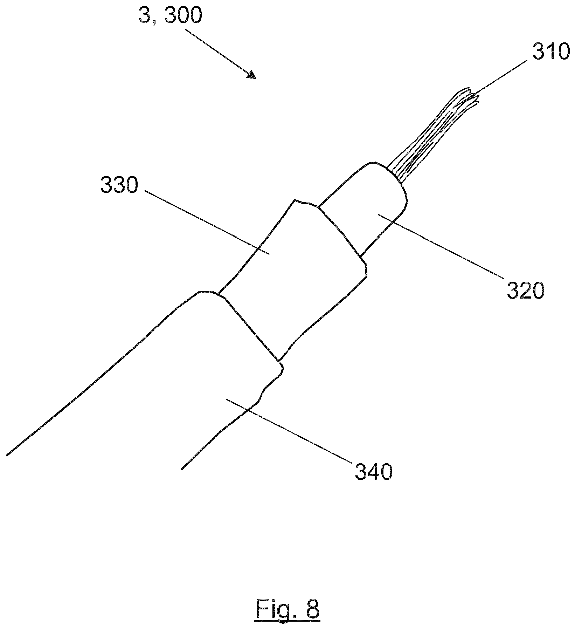

Figure 8 represents a schematic view of a multi-layered shielded cable (3, 300) of "coaxial type", according to a not claimed embodiment of the present invention.

As seen, the multi-layered shielded cable (3, 300) comprises:
- one conductive layer (310) electrically connected to the at least one electrode (2), made of conductive threads and/or textiles, for transporting the EEG signals to the electrical signal amplifier (A);
- one inner insulating layer (320) within which the conductive layer (310) is disposed, made of insulating threads and/or textiles, non-conductive polymers, or other means used in the textile industry;
- one shielding layer (330) within which the inner insulating layer (320) is disposed, made of conductive threads and/or textiles, for protecting the EEG signals from electrical and electromechanical interferences; and
- one outer insulating layer (340) within which the shielding layer (330) is disposed, made of insulating threads and/or textiles.

According to a specific example, embroidered conducting threads can be used to build this multi-layered shielded cable (3, 300) or coaxial cable, including a core corresponding to a conductive layer (310) made of conductive threads, an inner insulating layer (320) made of a dielectric, a shielding layer (330) made of a woven conductive shield or a mesh of conductive threads, and an outer insulating layer (340) made of a dielectric. Therefore, using textile and conductive threads, which can be easily embroidered, sewn or attached to the support (10). For example, this multi-layered shielded cable (3, 300) can be fixed on a textile support (10) by means of an upper and lower sewing thread, using a zig-zag stitch.

## Claims

1. A dry electroencephalogram measurement device, comprising a head support (10) on which the following components are attached and/or integrated:
- at least one electrode (2) for measuring EEG signals from a user (U); and
- at least one shielded cable (3), electrically connected to the at least one electrode (2), for transporting said EEG signals to an electrical signal amplifier (A);
wherein the support (10), the at least one electrode (2) and the at least one shielded cable (3) are integrally made with threads and/or textiles;
wherein the at least one shielded cable (3) is made of a combination of conductive and insulating threads and/or textiles, forming a multi-layered shielded cable (30) of laminate type, comprising:
- one conductive layer (31) electrically connected to the at least one electrode (2), made of conductive threads and/or textiles, for transporting the EEG signals to the electrical signal amplifier (A);
- two inner insulating layers (32) between which the conductive layer (31) is disposed, made of insulating threads and/or textiles, or non-conductive polymers; **characterized by**
- two shielding layers (33) between which the inner insulating layers (32) are disposed, made of conductive threads and/or textiles, for protecting the EEG signals from electrical and electromechanical interferences; and
- two outer insulating layers (34) between which the shielding layers (33) are disposed, made of insulating threads and/or textiles.

2. The device according to claim 1, **characterised in that** the at least one electrode (2) is made of conductive threads and/or textiles embroidered or sewn to the support (10), or attached to the same by heat sealing with thermoplastics polymers (5), such as polyvinyl chloride or polyurethane.

3. The device according to any of claims 1 to 2, **characterised in that** the at least one shielded cable (3) is embroidered or sewn to the support (10), or attached to the same by heat sealing with thermoplastics polymers, such as polyvinyl chloride or polyurethane.

4. The device according to any of claims 1 to 3, **characterised in that** the at least one electrode (2) and/or the at least one shielded cable (3) are made of non-woven fabrics, knitted fabrics, or woven fabrics.

5. The device according to any of claims 1 to 4, **characterised in that** the at least one electrode (2) and/or the at least one shielded cable (3) comprise a base of polyamide or polyester, plated or coated with conductive materials such as nickel, copper, or silver, tin, carbon, conductive polymers or combinations, among others.

6. A process for manufacturing a dry electroencephalogram measurement device, comprising the steps of:
a) providing or manufacturing a head support (10) made of textiles;
b) attaching or integrating to said support (10) at least one electrode (2) made of threads and/or textiles, for measuring EEG signals from a user (U); and
c) attaching or integrating to said support (10) at least one shielded cable (3) made of threads and/or textiles, electrically connected to the at least one electrode (2), for transporting said EEG signals to an electrical signal amplifier (A);
**characterised in that** the step c) comprises:
- providing or manufacturing a multi-layered shielded cable (30) of laminate type according to claim 1 made of a combination of conductive and insulating threads and/or textiles to form the at least one shielded cable (3); and
- sewing or embroidering the multi-layered shielded cable (30) to the support (10); or
- attaching the multi-layered shielded cable (30) to the support (10) by heat sealing with thermoplastics polymers (5), such as polyvinyl chloride or polyurethane.

7. The process according to claim 6, **characterised in that** the step b) comprises:
- embroidering one or more conductive threads to the textile support (10) to form the at least one electrode (2); or
- sewing a conductive textile to the textile support (10) to form the at least one electrode (2); or
- attaching one or more conductive threads and/or textiles to the textile support (10) by heat sealing with thermoplastics polymers (5), such as polyvinyl chloride or polyurethane, to form the at least one electrode (2).

## Patentansprüche

1. Vorrichtung zur Messung eines Trocken-Elektroenzephalogramms, umfassend eine Kopfstütze (10), auf welcher die folgenden Komponenten befestigt sind und/oder in welche sie integriert sind:
- mindestens eine Elektrode (2) zur Messung von EEG-Signalen aus einem Benutzer (U); und
- mindestens ein abgeschirmtes Kabel (3), welches mit der mindestens einen Elektrode (2) elektrisch verbunden ist, für den Transport der genannten EEG-Signale zu einem elektrischen Signalverstärker (A);
wobei die Stütze (10), die mindestens eine Elektrode (2) und das mindestens eine abgeschirmte Kabel (3) integral mit Fäden und/oder Textilien erstellt sind;
wobei das mindestens eine abgeschirmte Kabel (3) aus einer Kombination von leitfähigen und isolierenden Fäden und/oder Textilien erstellt ist, unter Bildung eines laminatartigen mehrschichtigen abgeschirmten Kabels (30), umfassend:
- eine leitfähige Schicht (31), welche mit der mindestens einen Elektrode (2) elektrisch verbunden ist, aus leitfähigen Fäden und/oder Textilien erstellt, für den Transport der EEG-Signale zum elektrischen Signalverstärker (A);
- zwei innere Isolierschichten (32), zwischen welchen die leitfähige Schicht (31) angeordnet ist, aus isolierenden Fäden und/oder Textilien, oder nicht leitfähigen Polymeren erstellt; **gekennzeichnet durch**
- zwei Abschirmschichten (33), zwischen welchen die inneren Isolierschichten (32) angeordnet sind, aus leitfähigen Fäden und/oder Textilien erstellt, für den Schutz der EEG-Signale gegen elektrische und elektromechanische Interferenzen; und
- zwei äußere Isolierschichten (34), zwischen welchen die Abschirmschichten (33) angeordnet sind, aus isolierenden Fäden und/oder Textilien erstellt.

2. Vorrichtung nach Anspruch 1, **dadurch gekennzeichnet, dass** die mindestens eine Elektrode (2) aus leitfähigen Fäden und/oder Textilien erstellt ist, welche an der Stütze (10) gestickt oder genäht sind, oder an derselben mittels Heißsiegelns mit thermoplastischen Polymeren (5), wie Polyvinylchlorid oder Polyurethan, befestigt sind.

3. Vorrichtung nach einem der Ansprüche 1 bis 2, **dadurch gekennzeichnet, dass** das mindestens eine abgeschirmte Kabel (3) an der Stütze (10) gestickt oder genäht ist, oder an derselben mittels Heißsiegelns mit thermoplastischen Polymeren, wie Polyvinylchlorid oder Polyurethan, befestigt ist.

4. Vorrichtung nach einem der Ansprüche 1 bis 3, **dadurch gekennzeichnet, dass** die mindestens eine Elektrode (2) und/oder das mindestens eine abgeschirmte Kabel (3) aus Vliesstoffen, Maschenwaren oder Geweben erstellt sind.

5. Vorrichtung nach einem der Ansprüche 1 bis 4, **dadurch gekennzeichnet, dass** die mindestens eine Elektrode (2) und/oder das mindestens eine abgeschirmte Kabel (3) eine Basis aus Polyamid oder Polyester umfassen, welche mit leitfähigen Materialien wie Nickel, Kupfer oder Silber, Zinn, Kohlenstoff, leitfähigen Polymeren oder Kombinationen, unter anderen, metallüberzogen oder beschichtet ist.

6. Verfahren zur Herstellung einer Vorrichtung zur Messung eines Trocken-Elektroenzephalogramms, welches die folgenden Schritte umfasst:
a) das Bereitstellen oder Herstellen einer Kopfstütze (10) erstellt aus Textilien;
b) das Befestigen an oder das Integrieren in die genannte Stütze (10) mindestens einer Elektrode (2) erstellt aus Fäden und/oder Textilien, zur Messung von EEG-Signalen aus einem Benutzer (U); und
c) das Befestigen an oder das Integrieren in die genannte Stütze (10) mindestens eines abgeschirmten Kabels (3) erstellt aus Fäden und/oder Textilien, welches mit der mindestens einen Elektrode (2) elektrisch verbunden ist, für den Transport der genannten EEG-Signale zu einem elektrischen Signalverstärker (A);
**dadurch gekennzeichnet, dass** der Schritt c) Folgendes umfasst:
- das Bereitstellen oder Herstellen eines laminatartigen mehrschichtigen abgeschirmten Kabels (30) nach Anspruch 1 erstellt aus einer Kombination von leitfähigen und isolierenden Fäden und/oder Textilien, um das mindestens eine abgeschirmte Kabel (3) zu bilden; und
- das Nähen oder Sticken des mehrschichtigen abgeschirmten Kabels (30) an der Stütze (10); oder
- das Befestigen des mehrschichtigen abgeschirmten Kabels (30) an der Stütze (10) mittels Heißsiegelns mit thermoplastischen Polymeren (5), wie Polyvinylchlorid oder Polyurethan.

7. Verfahren nach Anspruch 6, **dadurch gekennzeichnet, dass** der Schritt b) Folgendes umfasst:
- das Sticken eines oder mehrerer leitfähigen Fäden an der Textilstütze (10), um die mindestens eine Elektrode (2) zu bilden; oder
- das Nähen eines leitfähigen Textils an der Textilstütze (10), um die mindestens eine Elektrode (2) zu bilden; oder
- das Befestigen einer oder mehrerer leitfähigen Fäden und/oder Textilien an der Textilstütze (10) mittels Heißsiegelns mit thermoplastischen Polymeren (5), wie Polyvinylchlorid oder Polyurethan, um die mindestens eine Elektrode (2) zu bilden.

## Revendications

1. Dispositif de mesure d'électroencéphalogramme à sec, comprenant un support de tête (10) sur lequel les composants suivants sont fixés et/ou intégrés :
- au moins une électrode (2) destinée à mesurer des signaux EEG provenant d'un utilisateur (U) ; et
- au moins un câble blindé (3), électriquement raccordé à ladite au moins une électrode (2), destiné à transporter lesdits signaux EEG vers un amplificateur de signal électrique (A) ;
dans lequel le support (10), ladite au moins une électrode (2) et ledit au moins un câble blindé (3) sont réalisés d'une seule pièce à l'aide de fils et/ou de textiles ;
dans lequel ledit au moins un câble blindé (3) est réalisé à partir d'une combinaison de fils et/ou de textiles conducteurs et isolants, formant un câble blindé multicouche (30) de type stratifié, comprenant :
- une couche conductrice (31) électriquement raccordée à ladite au moins une électrode (2), réalisée en fils et/ou textiles conducteurs, destinée à transporter les signaux EEG vers l'amplificateur de signal électrique (A) ;
- deux couches isolantes internes (32) entre lesquelles est disposée la couche conductrice (31), réalisées en fils et/ou textiles isolants, ou en polymères non conducteurs ;
**caractérisé en ce qu'**il comprend :
- deux couches de blindage (33) entre lesquelles sont disposées les couches isolantes internes (32), réalisées en fils et/ou textiles conducteurs, destinées à protéger les signaux EEG contre les interférences électriques et électromécaniques ; et
- deux couches isolantes externes (34) entre lesquelles sont disposées les couches de blindage (33), réalisées en fils et/ou textiles isolants.

2. Dispositif selon la revendication 1, **caractérisé en ce que** ladite au moins une électrode (2) est réalisée en fils et/ou textiles conducteurs brodés ou cousus sur le support (10), ou fixée à celui-ci par thermoscellage au moyen de polymères thermoplastiques (5), tels que le polychlorure de vinyle ou le polyuréthane.

3. Dispositif selon l'une quelconque des revendications 1 à 2, **caractérisé en ce que** ledit au moins un câble blindé (3) est brodé ou cousu sur le support (10), ou fixé à celui-ci par thermoscellage au moyen de polymères thermoplastiques, tels que le polychlorure de vinyle ou le polyuréthane.

4. Dispositif selon l'une quelconque des revendications 1 à 3, **caractérisé en ce que** ladite au moins une électrode (2) et/ou ledit au moins un câble blindé (3) sont réalisés en non-tissés, en étoffes tricotées ou en étoffes tissées.

5. Dispositif selon l'une quelconque des revendications 1 à 4, **caractérisé en ce que** ladite au moins une électrode (2) et/ou ledit au moins un câble blindé (3) comprennent une base en polyamide ou en polyester, plaquée ou revêtue de matériaux conducteurs tels que le nickel, le cuivre, l'argent, l'étain, le carbone, des polymères conducteurs ou des combinaisons de ceux-ci, entre autres.

6. Procédé de fabrication d'un dispositif de mesure d'électroencéphalogramme à sec, comprenant les étapes consistant à :
a) fournir ou fabriquer un support de tête (10) réalisé en textiles ;
b) fixer ou intégrer audit support (10) au moins une électrode (2) réalisée en fils et/ou textiles, destinée à mesurer des signaux EEG provenant d'un utilisateur (U) ; et
c) fixer ou intégrer audit support (10) au moins un câble blindé (3) réalisé en fils et/ou textiles, électriquement raccordé à ladite au moins une électrode (2), destiné à transporter lesdits signaux EEG vers un amplificateur de signal électrique (A) ;
**caractérisé en ce que l'étape c)** comprend :
- fournir ou fabriquer un câble blindé multicouche (30) de type stratifié selon la revendication 1, réalisé à partir d'une combinaison de fils et/ou de textiles conducteurs et isolants, de manière à former ledit au moins un câble blindé (3) ; et
- coudre ou broder le câble blindé multicouche (30) sur le support (10) ; ou
- fixer le câble blindé multicouche (30) au support (10) par thermoscellage au moyen de polymères thermoplastiques (5), tels que le polychlorure de vinyle ou le polyuréthane.

7. Procédé selon la revendication 6, **caractérisé en ce que** l'étape b) comprend :
- broder un ou plusieurs fils conducteurs sur le support textile (10) afin de former ladite au moins une électrode (2) ; ou
- coudre un textile conducteur sur le support textile (10) afin de former ladite au moins une électrode (2) ; ou
- fixer un ou plusieurs fils et/ou textiles conducteurs sur le support textile (10) par thermoscellage au moyen de polymères thermoplastiques (5), tels que le polychlorure de vinyle ou le polyuréthane, afin de former ladite au moins une électrode (2).
